# EUROPEAN PATENT SPECIFICATION

(11) **EP 3 429 660 B1**
(45) Date of publication and mention of the grant of the patent: **25.01.2023**
(21) Application number: 17709938.9
(22) Date of filing: 08.03.2017
(51) Int. Cl.: A61M 5/142, F04B 19/00, F04B 43/04, H01J 37/08, G01N 33/487

(54) **ION CONDUCTIVE DEVICE WITH CONTROLLED DELIVERY ELECTRODE**
IONENLEITENDE VORRICHTUNG MIT GESTEUERTER ABGABEELEKTRODE
DISPOSITIF CONDUCTEUR D'IONS AVEC ÉLECTRODE POUR LIVRAISON CONTRÔLÉE

(30) Priority: 15.03.2016 SE 1650348
(43) Date of publication of application: 23.01.2019
(73) Proprietor: OBOE IPR AB, 581 18 Linköping (SE)
(72) Inventor: JONSSON, Amanda, 586 66 Linköping (SE); ARBRING SJÖSTRÖM, Theresia, 587 58 Linköping (SE); SIMON, Daniel, 582 25 Linköping (SE); BERGGREN, Magnus, 602 18 Norrköping (SE)
(74) Representative: AWA Sweden AB
(86) International application number: PCT/EP2017/055385
(87) International publication number: WO 2017/157729

(56) References cited:
- EP-A1- 1 862 799
- WO-A1-2005/053836
- WO-A1-2009/115103
- US-A1- 2010 255 557
- ISAKSSON JOAKIM ET AL: "Electronic control of Ca2+ signalling in neuronal cells using an organic electronic ion pump", NATURE MATERIALS, NATURE PUBLISHING GROUP, GB, vol. 6, no. 9, September 2007 (2007-09), pages 673-679, XP002482909, ISSN: 1476-1122, DOI: 10.1038/NMAT1963 [retrieved on 2007-07-22]
- A. JONSSON ET AL: "Therapy using implanted organic bioelectronics", SCIENCE ADVANCES, vol. 1, no. 4, 8 May 2015 (2015-05-08), pages e1500039-e1500039, XP055372820, DOI: 10.1126/sciadv.1500039
- Daniel T Simon ET AL: "Precise Neurotransmitter-Mediated Communication with Neurons In Vitro and In Vivo Using Organic Electronics", Journal of Biomechanical Science and Engineering, January 2010 (2010-01), pages 208-217, XP055373148, DOI: 10.1299/jbse.5.208 Retrieved from the Internet: URL:https://www.jstage.jst.go.jp/article/j bse/5/3/5_3_208/_pdf [retrieved on 2017-05-16]
- A. JONSSON ET AL: "Chemical delivery array with millisecond neurotransmitter release", SCIENCE ADVANCES, vol. 2, no. 11, 2 November 2016 (2016-11-02), pages e1601340-e1601340, XP055372812, DOI: 10.1126/sciadv.1601340

## Description

### Technical field

The present invention relates to an ion conductive device for a controlled delivery of ions to a target electrolyte such as tissue, body fluids or cells, and more specifically to enhanced spatial and temporal delivery in vitro.

### Background

Drugs typically have their therapeutic action at specific sites in the body, but are often administered systemically. This means that only a small portion of the drug ends up where it is needed, and the rest may cause side effects elsewhere in the body. By delivering drugs locally, where and when they are needed, a much lower dose can be used, and hence side effects may be avoided. Indeed, many drugs that today fail in clinical trials because of their adverse effects due to high dosages could in fact be effective and without side effects if they were delivered locally and at very low doses.

Neurological disorders, such as Parkinson's disease and epilepsy result from perturbations to the nervous system. Treatments for neurological disorders include administration of pharmaceuticals and electrical stimulation of the nerves. With electrical stimulation a very fast effect is achieved, but the effect cannot target a certain nerve signaling pathway but rather effects all nearby nerve cells. Pharmaceutical treatment, on the other hand, is more specific, but much slower since the drugs need to travel to the site of action.

A drug delivery device, such as an implantable device that could release drugs locally and on a millisecond timescale would have the benefits of specificity that pharmaceutical treatment has, along with the fast response typical of electrical stimulation. Also, the side effects due to high dosage that is associated with systemic administration of drugs, as well as side effects associated with electrical stimulation, such as muscle twitching and sensation, could be avoided.

To be a good candidate for an implanted drug delivery device, the technology should exhibit a few important features. For many applications it is desirable that the delivery rate can be controlled in time, so that the drug is delivered only when it is needed, i.e. the passive leakage should be low, meaning that the device should exhibit a good ON/OFF function. Depending on the application, it may be essential that the drugs can be delivered quickly when needed. This is specifically important in neurological disorders or in general when interacting with the nervous system. For example in neuronal prostheses e.g. human-made electrical/technological connections to neurons or neural tissue where the speed is essential. Neurons generally communicate with one another, and with their target tissue e.g. neuromuscular junctions, by transferring chemical signals e.g. via neurotransmitters, in bursts lasting for ~10 milliseconds. For example, the fine motor motion of the hands and fingers requires this high speed ON/OFF chemical signaling; and a slower signaling could result, in e.g. continuous muscle contraction.

Furthermore, it is essential that any implantable device technology have a long lifetime. This means that the device should be stable inside the body, not produce inflammation, and that enough drugs/therapeutic/signaling substances can be stored in the device. The latter requirement typically implies that a reservoir needs to be coupled to the device.

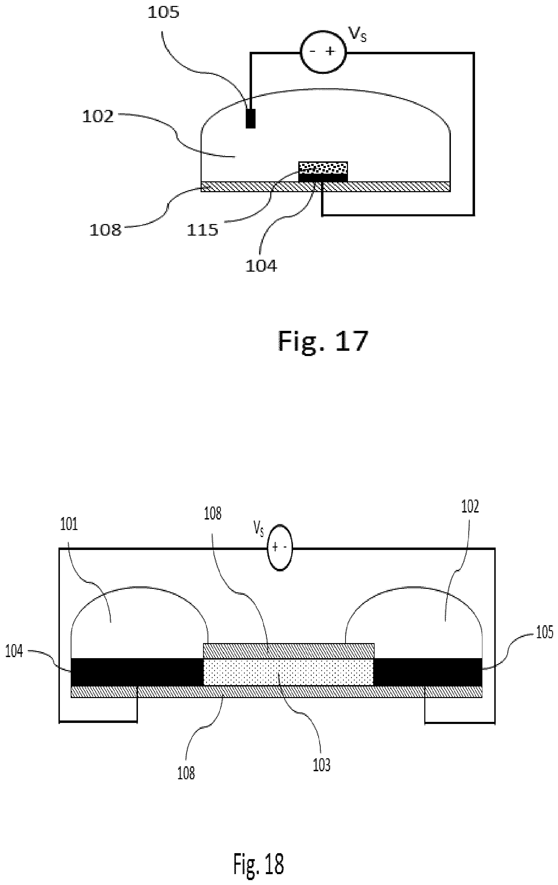

It is of interest to control the amount of a drug that is released at several sites in the same fluid individually (each release site, or drug outlet, can be ON or OFF independently from the others). As an example, a prosthetic device that aims at connecting an arm with the brain (where the original neuronal connections have been lost) may need to activate or inhibit hundreds or thousands of different regions or nerve/muscle cells to achieve proper movements of the arm. The body fluid contains a significant quantity of mobile ions, and is therefore ionically conducting. Therefore, it constitutes an electrolyte by definition, and is herein also referenced as a target electrolyte. Prior art devices such as the Organic Electronic Ion Pump (OEIP, see Fig. 18) delivers ions from a channel to a target. This pump must be combined with several other pumps to achieve several delivery points. Such a combined device would be large, and with many components that it would be difficult to maintain sufficient robustness for implant applications.

A technology with a single source electrolyte/reservoir is significantly more desirable from both a practical perspective and based on eventual production costs.

Likewise, a technology in which the addressability function of multiple, independent ON/OFF delivery sites does not add significant bulkiness or complexity is advantageous from the perspectives of practicality, e.g., of production and deployment, cost, and possibly even regulatory issues, e.g., specific requirements for medical implants.

There are several methods for local drug delivery already in use, including implanted pumps where the delivery rate can be controlled in time. When the drug is dissolved and delivered in a carrier fluid this dilutes the environment where the drug is delivered, and can lead to an increased pressure if the drug is delivered into a confined compartment. Microfluidics is the scaled down version of drug delivery in fluids, mostly used for in vitro lab-on-a-chip applications. Even though the volumes are much smaller, the same problem with increased pressure still exists. Furthermore, the amount of delivered drug is not controlled to a very high extent for either of these fluidic techniques. Other techniques used in practice are transdermal patches and subdermal implants that exhibit passive delivery, meaning that drugs are continuously released at a predetermined rate. The delivery rate can thus not be actively controlled in time with a sufficiently high degree of precision.

A few techniques for local drug delivery utilize the fact that many drugs and neurotransmitters are, or can occur in, electrically charged form. This implies that they can be controlled and measured electrically. These techniques include drug release from conducting polymers and iontophoresis. Iontophoresis, or electromotive drug administration (EMDA), is a method for administering charged drugs through the skin with an applied electric field. This method is not very exact in terms of the amount of delivered drugs. Charged drugs have also been incorporated as counter ions into conducting polymers, and when the charge of the polymer is altered as a function of oxidation or reduction, the drug (counter ions) is expelled and released from the conducting polymer without any liquid flow (as shown in Fig. 17). Although many research groups have successfully used this principle, it suffers from high passive leakage, since ions of the electrolyte/body fluid are passively exchanged with the ionic drugs loaded in the conducting polymer, regardless of the addressing voltage. Furthermore, only the drugs originally incorporated into the conducting polymer during the fabrication or pre-usage phase can be released, which limits the amount of drug that can be delivered.

The OEIP is an alternative solution to deliver charged drugs/biomolecules from micrometer-sized outlets without any significant fluid flow. The delivery rate of drugs from an OEIP is controlled by the applied current, and can thus be tuned.

With OEIPs, several outlets in the same target can be separately addressed if they all have separate source electrolytes and hence separate channels. One source electrolyte can also be used to address several outlets if each outlet is located in a separate target electrolyte. As described in EP2068146 several target electrolytes may be used, since a waste electrolyte is introduced into the system. However, if the device is to be implanted, the situation with multiple target electrolytes is not relevant at all, because then there is only one target electrolyte, e.g. the body fluid.

One design of the OEIP enables millisecond-fast delivery, by making a small, high aspect- ratio hole, i.e. high height to diameter ratio, to the target electrolyte so that delivery occurs out of the plane instead of in the plane of thin films (100 nm scale). To increase the ON/OFF ratio during operation of this device, an ionic bipolar membrane diode (to be explained in the following description) is incorporated, allowing only one direction of the ionic current through the target outlet.

However, the OEIP in any form, such as for instance described in EP1862799, WO2009/115103 A1, US 8715478B2, and WO2010118754 cannot be used to construct several individually controlled outlets in the same electrolyte if a common source electrolyte is used. The reason why this is not possible is because the electrical potential of the electrolytes (the source, waste and target electrolytes) control the delivery currents and cannot distinguish between the different outlets. Therefore, if the geometry with several outlets is used with OEIPs, the outlets will all be ON or all be OFF at the same time.

There is thus a need for an ion transport device, which is preferably an implantable device, where it is possible to individually control delivery of charged chemical species through one or several delivery sites in a common target electrolyte.

### Summary

It is an object of the present disclosure, to provide an improved ion conductive device, which eliminates or alleviates at least some of the disadvantages of the prior art devices.

The invention is defined by the appended independent claims. Embodiments are set forth in the appended dependent claims and in the following description and drawings.

According to a first aspect, there is provided a device comprising a first electrode provided at or in a source electrolyte, and at least one ion conductive channel, wherein said first electrode is arranged at a first portion of the ion conductive channel, and a second electrode provided in a target electrolyte, wherein said target electrolyte is arranged at a second portion of the ion conductive channel, and wherein said first and second electrodes provides an electrical control of an ion flow through the ion conductive channel, wherein the device further comprises at least one controlled delivery electrode (CDE) arranged adjacent to or in the second portion of the ion conductive channel, wherein said first and second electrodes further provides an electrical control of an ion flow through the controlled delivery electrode to the target electrolyte, and wherein said controlled delivery electrode is adapted to deliver ions from said ion conductive channel to said target electrolyte, and wherein said controlled delivery electrode comprising an electronically and ionically conductive material and an electrical contact, wherein said controlled delivery electrode is arranged in ionic contact with, and between, said ion conductive channel and the target electrolyte, wherein said electrical contact provides for an electrical control potential over the controlled delivery electrode to control an ion flow between the controlled delivery electrode and the target electrolyte.

The device is thus based on the previously disclosed Organic Electronic Ion Pump (OEIP), however the inventive device comprises at least three electrodes, i.e. the source, target and the at least one controlled delivery electrode. Each controlled delivery electrode is arranged between its electrical connection and its outlet, hence forming an ON/OFF switch enabling delivery and preventing leakage by controlling the voltage of the controlled delivery electrode with respect to the target electrolyte. Because there is electronical conductivity in the controlled delivery electrode, very close to the release site, ions can be released faster, i.e. with a fast switch-on speed, in the millisecond range, from the application of a control potential. This electrical switch-on is much faster compared to the switch-on speed of previous OEIP (seconds). The controlled delivery electrode thus forms an active component for achieving a faster release of ions both from the controlled delivery electrode itself and from the ion conductive channel into the target electrolyte.

In contrast to other types of electronically and ionically conductive materials for delivery of ions, such as drugs, to a specific target electrolyte, e.g. a tissue or a nerve cell, the controlled delivery electrode, which may also serve as a storage of ions, may be continually refilled with ions from the source electrolyte through the ion conductive channel. This may abolish the need for an additional reservoir to be connected to the device. This may in turn allow for the creation of very small devices, for instance as smaller adhesive patches to be applied on a patient or in connection with nerves, brain or heart.

This provides for an implantable device which can be used for continuously providing medicaments to a patient, as well as for deploying a medicament quickly when the need occurs, e.g. in a pulsed manner, but which does not need to be removed in order to be refilled. The electronically and ionically conductive material in the controlled delivery electrode may thus serve as a pre-loaded reservoir for ions, to be deployed into the target electrolyte at a specific time.

The size of the controlled delivery electrode and its hole or interface to the target electrolyte may be adapted for the desired rate of discharge needed, i.e. a larger controlled delivery electrode and a larger hole provides a faster discharge of ions into the target electrolyte.

The electronically and ionically conductive material of the controlled delivery electrode may be permselective for either cationic or anionic species.

The material of the controlled delivery electrode may thus be adapted depending on the type of ions to be delivered. The controlled delivery electrode is thus electronically as well as cationically (or anionically) conductive.

The electronically and ionically conductive material may include a conducting polymer such as PEDOT.

The electronically and ionically conductive material may be adapted for a charge being injected or extracted from the material.

This provides for a material having a sufficiently high capacitance (for being able to store ion(s).

The device may comprise at least two controlled delivery electrodes, and said at least two controlled delivery electrodes may be separated from each other with a ion conductive channel having a finite ion conductivity, to control ion flow from the ion conductive channel into the target electrolyte from each controlled delivery electrode separately.

The at least two controlled delivery electrodes thus provide at least two spatially different delivery sites or outlets in said target electrolyte, which can be individually controlled through the activation of the controlled delivery electrodes. This provides for a device which only has one source electrolyte, but where ions can be delivered at different locations in the target electrolyte. As the controlled delivery electrodes can be individually addressed it is possible to precisely control the delivery to specific delivery sites or outlets, or for instance cover a larger area, without having to use several different devices. The device may thus comprise at least four electrodes, i.e. a source and target electrode and at least two controlled delivery electrodes, controlling a respective outlet. Basically, by controlling the electric potentials at the controlled delivery electrodes for each outlet, the ion delivery at multiple sites in a target electrolyte can be controlled individually, although the potentials of the source, target and waste electrolytes are kept constant.

The resistance between the controlled delivery electrode and the target electrolyte is preferably sufficiently high to enable the potential drop between the contact of the controlled delivery electrode and the target electrolyte.

The controlled delivery electrodes may be arranged on or in the same or different ion conductive channel and the controlled delivery electrodes may further be separated from each other with an ion conductive channel material having a finite ion conductivity.

By "finite ion conductivity" it is meant that the material has a sufficiently high ionic resistance (ion conductive channel) to prevent so called crosstalk between outlets or the controlled delivery electrodes.

A barrier layer or ion barrier may be arranged between the controlled delivery electrode and the target electrolyte.

The ion barrier thus forms an ion diode together with the electronically and ionically conductive material in the controlled delivery electrode. The barrier provides a way of stopping passive leakage from the controlled delivery electrode and the ion conductive channel to the target electrolyte especially when combined with a small ionic current from the target electrolyte to the controlled delivery electrode.

The barrier or ion barrier may comprise a material having a fixed concentration of opposite charges with respect to the fixed charges of the electronically and ionically conductive material of the controlled delivery electrode.

The ion barrier thus provides an ion current rectification together with the electronically and ionically conductive material in the controlled delivery electrode, since the two together form an ionic bipolar membrane diode. The barrier is not electronically conducting, but is merely a polycation or polyanion material (or any other porous material with fixed charges opposite to the electronically and ionically conductive material).

The barrier may alternatively geometrically restrain ion flow.

This may e.g. be a narrow passage or similar, which together with the permselectivity of the controlled delivery electrode and ion conductive channel materials results in ion current rectification. Hence it forms another type of ion diode.

The device may further comprise at least two ion conductive channels, and multiple controlled delivery electrodes may be arranged in or on said multiple ion conductive channels, wherein said controlled delivery electrodes may be in ionic contact with the same target electrolyte, and the controlled delivery electrodes may be separated from each other by an ion conductive channel having a finite ion conductivity.

This provides for an even more versatile device in which there may be several delivery sites or outlets into the same target electrolyte, while still having only one source electrolyte.

The device may comprise at least one waste channel, and each waste channel may comprise a waste electrolyte and a waste electrode.

According to a second aspect there is provided a method of operating a device according to the first aspect, comprising the steps of:
a) providing a source electrolyte comprising the ions to be transported,
b) providing a target electrolyte to where the ions are transported,
c) bringing the source electrode of the device in contact with the source electrolyte,
d) optionally providing a waste and a waste electrolyte;
e) bringing the target electrode in contact with the target electrolyte
f) applying a potential to the source electrode, the target electrode and the controlled delivery electrode, and optionally to the waste electrode, effecting ion transport from the source electrolyte, to the controlled delivery electrode, or optionally to the waste electrolyte; and
g) altering the potential of the controlled delivery electrode to switch on ion transport to the target electrolyte.

According to a third aspect there is provided the use of a device according to the first aspect, for delivering ions from a source electrolyte to a target electrolyte through a controlled delivery electrode.

Said target electrolyte may comprise any one of tissue, body fluids or cells in vitro.

### Brief Description of the Drawings

Embodiments of the present solution will now be described, by way of example, with reference to the accompanying schematic drawings.
Fig. 1 is a schematic side view of the device comprising a single ion conductive channel and a single controlled delivery electrode device.
Fig. 2 is a schematic side view of the device with an enlarged controlled delivery electrode.
Fig. 3 is a schematic side view of the device with a single ion conductive channel and multiple controlled delivery electrodes placed in series.
Fig. 4 is a schematic side view of the device with multiple ion conductive channels, each having a controlled delivery electrode.
Fig. 5 is a schematic side view of a controlled delivery electrode with a barrier layer.
Fig. 6 is a schematic side view of a controlled delivery electrode with a barrier based on geometry.
Fig. 7 is a schematic side view of the device having multiple ion conductive channels and multiple controlled delivery electrodes.
Fig. 8 is a schematic top view of the device having a controlled delivery electrode, with multiple source electrolytes and a single target electrolyte.
Fig. 9 is a schematic top view of the device having controlled delivery electrodes, with a single source electrolyte and multiple target electrolytes.
Fig. 10 is a schematic top view of the device having multiple controlled delivery electrodes provided in parallel in the ion conductive channel.
Fig. 11 is a schematic side view of the device comprising a controlled delivery electrode and a separate waste electrolyte.
Fig. 12a is a schematic graph showing an aproximate potential path through the barrier.
Fig. 12b is a schematic graph showing an aproximate potential path through controlled delivery electrode to the waste.
Fig. 13a shows a graph of a delivery pulse.
Fig. 13b show that the target current is the sum of the source, waste and controlled delivery electrode currents.
Fig. 14 is a graph showing delivery levels and delivery response time for a standard ion pump OEIP according to the dotted line and the solid line according to the invention having a controlled delivery electrode.
Fig. 15 is a schematic side view of a device system having one source electrolyte and two encapsulated hoses/pipe-like struktures which both are connected to a common waste electrolyte. The device having multiple ion conductive channels, each provided with one or more barrier equipped controlled delivery electrodes which are all in connection with a common target electrolyte.
Fig. 16 shows a schematic top view of a device system having three ion conductive channels, each provided with a barrier equipped controlled delivery electrode in connection with a target electrolyte and each channel in connection to a common source electrolyte and a common waste electrolyte.
Fig. 17 is a schematic side view of a prior art charged drug device.
Fig. 18 is a schematic side view of the prior art Organic Electronic Ion Pump (OEIP).

### Description of Embodiments

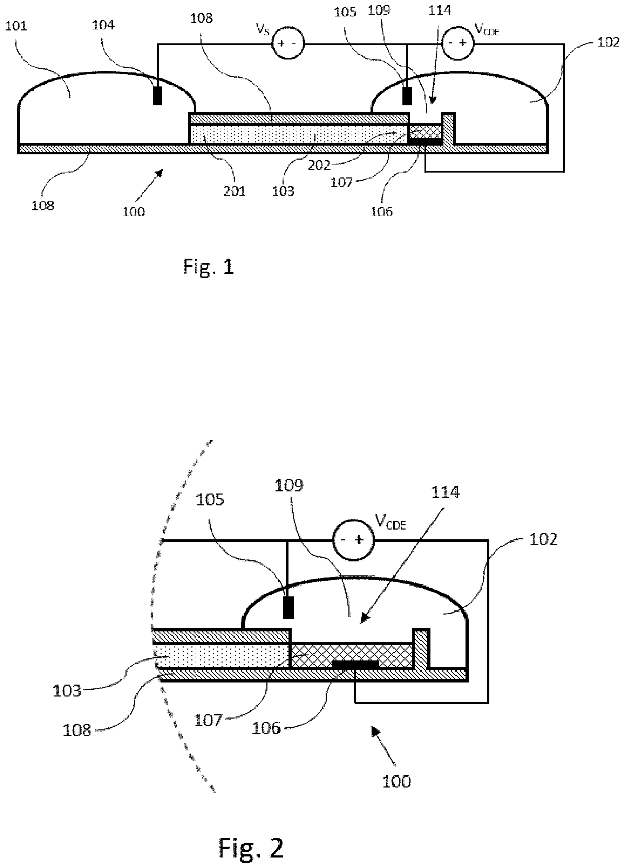

Fig. 1 shows a device 100 or system according to the invention describing the source electrolyte 101 comprising a first electrode 104, also denoted source electrode, a target electrolyte 102 which includes a second electrode 105, also denoted target electrode and a ion conductive channel 103 having a first end 201 and a second end 202.

The device 100 or system can either be constructed as a polyanion system or a polycation system depending on the fixed charges of the polymer of the permselective ion conductive channel.

The ion conductive channel 103 should be designed or treated in such a way as to minimize/reduce electronical conductivity, but preserve or provide ionic conductivity, e.g. by a polystyrene sulfonate(PSS)-derivate or doping.

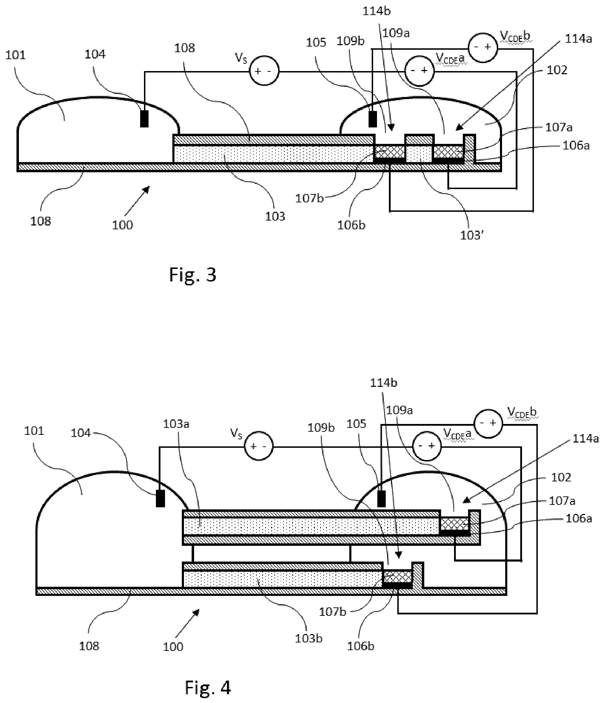

Each respective controlled delivery electrode 114, 114a, 114b, see e.g. Fig. 1, Fig. 3, Fig. 4 or Fig. 10, comprises polymers having both electronic and ionic conductivity, connected to an electrical contact 106, 106a, 106b. The controlled delivery electrodes are separated by a portion of the ion conductive channel 103', 103a', 103b', see Fig. 3, Fig. 7 or Fig. 15.

The ion conductive channel 103 and the controlled delivery electrodes 114, 114a, 114b may be insulated from the electrolytes through an electronically and ionically insulating layer 108, which also minimizes transport of water. On a plane surface, this insulation may consist of a wall, or in tissue, of pipe-like structure with openings through the insulation layer for the respective controlled delivery electrode.

It is preferred that the potential Vₛ over the source electrode and the target electrode is kept at a constant value. The ion conductive channel can then be filled with ions by creating a potential over the channel e.g. by making a difference in potential V_{CDE}a and V_{CDE}b between the respective controlled delivery electrode and the target electrode see Fig. 3 for a given constant source electrode potential. If for example, the source electrode has a potential of 10 volt, the target electrode 0 volt and the electrical contact of a controlled delivery electrode -0.4 volt, then positive ions will be collected at that controlled delivery electrode.

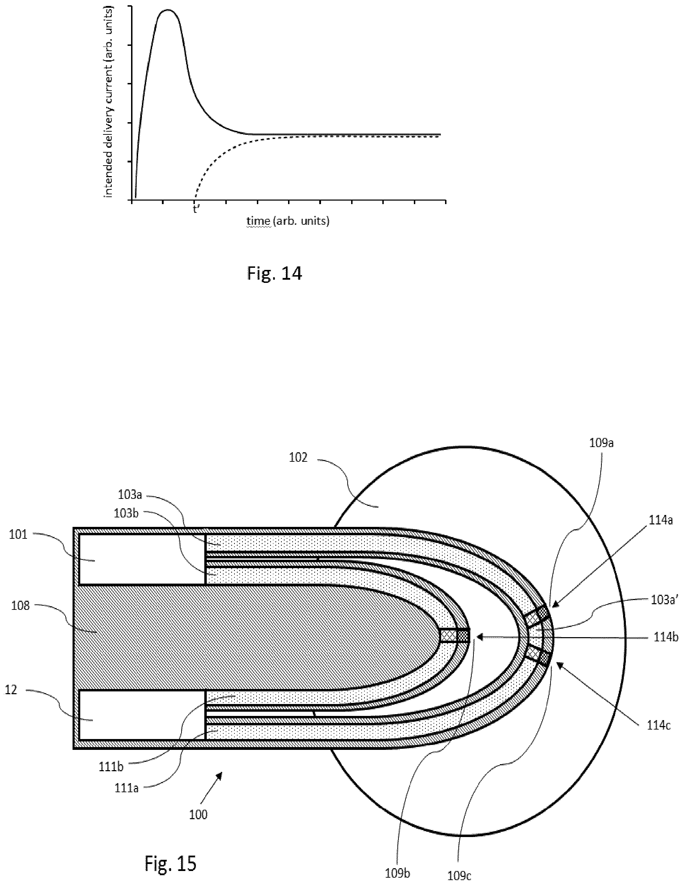

To achieve delivery of ions, the electrical contact 106 of a controlled delivery electrode is switched from negative to positive potential versus the target electrode 105. This will make the positive ions move into the target electrolyte. By quickly altering the internal state of the electronically and ionically conductive material 107, a burst of ions will be quickly repelled from the electronically and ionically conductive material 107 and delivered to the target electrolyte. The electronically and ionically conductive material 107 can be enlarged as seen in Fig. 2, to be able to increase the burst of ions see Fig. 14. Hence a larged and well defined amount of ions or drugs can be quickly delivered into the target electrolyte 102.

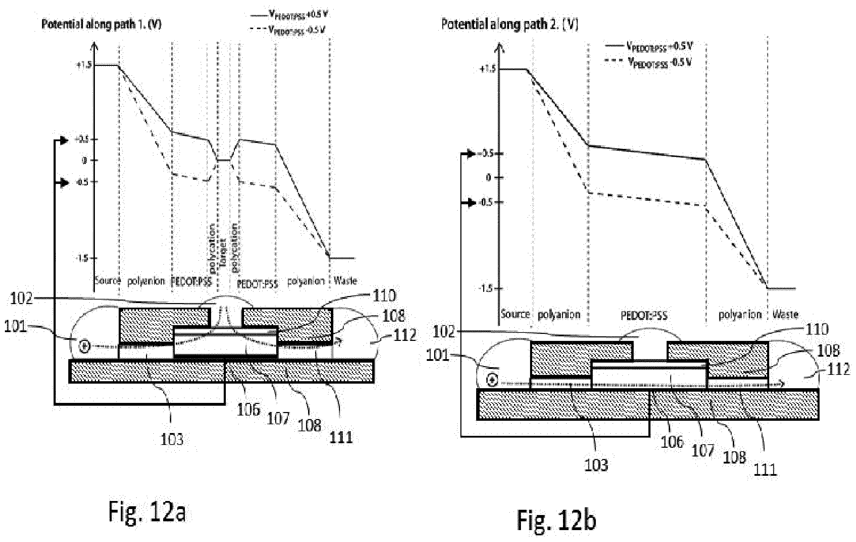

Fig. 12, Fig. 13 and Fig. 14 show the potential profile and currents at different parts of the device 100 when the control voltage is applied. Fig. 12a and Fig. 12b show approximate potential profiles along two pathways in the device. Fig. 12a shows the potentials ions would experience in travelling from the source electrolyte to the target electrolyte and to the waste electrolyte. A controlled delivery electrode that is ON will have a negative potential gradient from the controlled delivery electrode 114 through the barrier 110 to the target electrolyte, meaning that the diode is forward biased, which makes cations move from the electronically and ionically conductive material 107 to the target electrolyte and anions from the target electrolyte into the electronically and ionically conductive material 107. For a controlled delivery electrode that is OFF, the potential gradient from the electronically and ionically conductive material 107 to the target electrolyte is instead positive, which attracts cations toward the electronically and ionically conductive material 107 and cations toward the polycation in polyanion/polycation interface, thus the diode is reverse-biased and the interface is depleted of ions, leading to a low current.

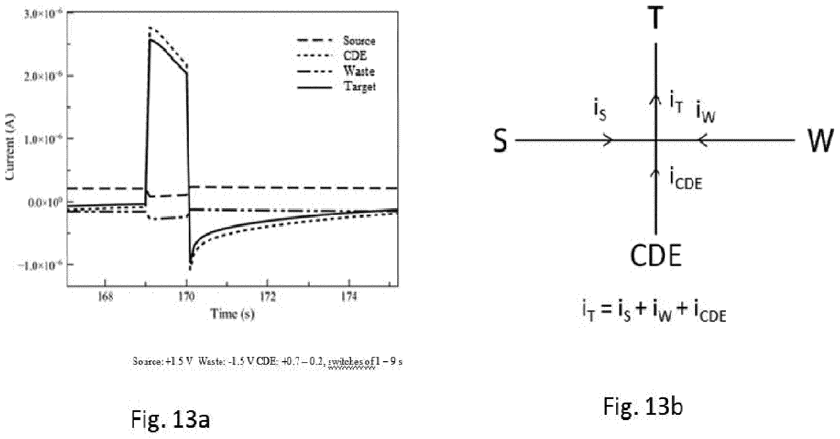

Fig. 13 shows measured currents i_{Source}, i_{Waste}, i_{CDE} and the calculated i_{Target} when switching from reverse to forward bias in a one second long delivery pulse (i_{Target} = i_{Source} + i_{Waste} + i_{CDE}) from a single controlled delivery electrode. The voltage of the electronically and ionically conductive material 107 is switched between ±0.5 V, relative to the target potential. A diode behavior can be noted (high target current in forward bias, and low in reverse bias). In reverse bias, the controlled delivery electrode is refilled with ions from the source.

Fig. 14 shows the current of intended molecules to be delivered, not the overall current. For example, if a substance in a prior art ion pump (dashed line) is to be delivered, there will be a delay of t' before the molecule makes it to the delivery site. Even if the channel is prefilled, there will still be a rise time due to some passive diffusion at the very tip (i.e., same dashed curve shape even if t'=0). The solid line is the "burst" achieved from a controlled delivery electrode according to the invention.

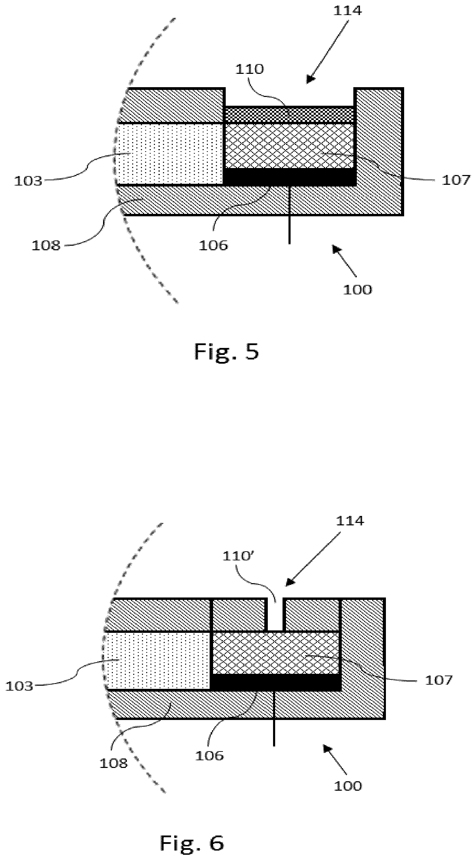

A physical or functional barrier layer 110 as illustrated in Fig. 5, improves the functionality by allowing individual addressing with highly reduced leakage.

The barrier may be based on an oppositely charged material, compared to the charge of the permselective ion conductive channel and the net charge of the material in the controlled delivery electrode.

The barrier 110' may also comprise a small-area hole, as illustrated by Fig. 6, or a hole with a high height-to-area aspect ratio. In either case, the effect of the barrier or barrier layer is to minimize the reverse current needed to prevent leakage, which prevents local depletion of cations at the delivery site. The barrier can also be constructed using previously known ionic diodes.

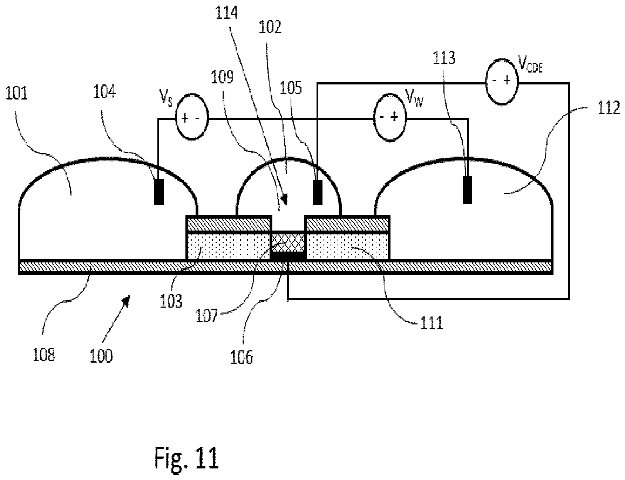

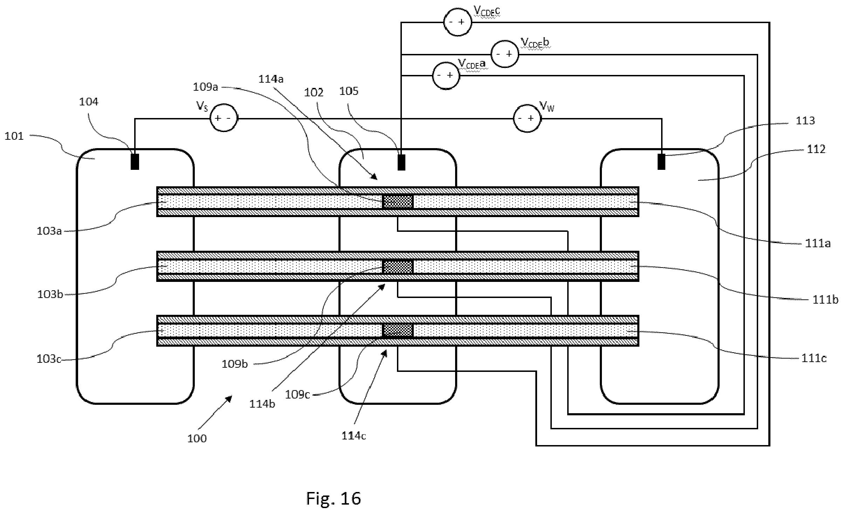

The device 100 or device system can be extended to include a waste portion comprising a waste channel 111 leading to a waste electrolyte 112 and a waste electrode 113, see as illustarted in Fig. 11, Fig.15 or Fig. 16. The waste is used to carry away ions and to provide the means of a constant flow of ions through the ion conductive channels and controlled delivery electrodes. This facilitates the filling of the ion conductive channel. The waste thus increases the speed with which the controlled delivery electrode is filled with ions.

When an alternating current is applied as V_{CDE} a serial burst effect is achieved.

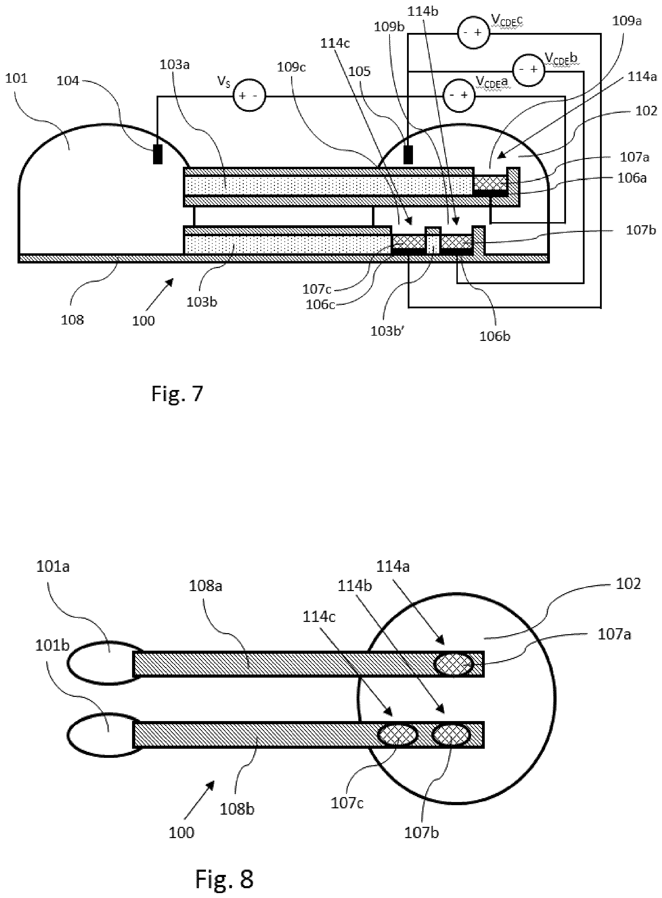

The device 100 or system may be used with a common target electrolyte but different source electrolytes as illustated in Fig. 8, and where the different source electrolytes include different substances or medical drugs to be delivered.

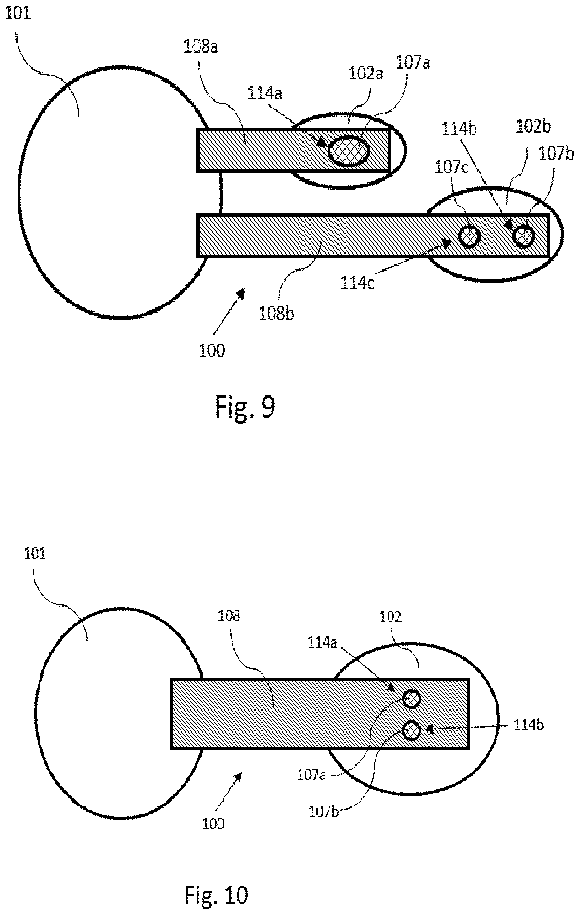

Alternatively, the device 100 or system can be used with a common source electrolyte but with different target electrolytes, see Fig. 9, and where the target electrolytes are located at different places to receive the same type of substance or medical drug.

Fig. 17 shows a schematic side view of a prior art charged drug device. Charged drugs have been incorporated as counter ions into conducting polymers. The drug (counter ions) is expelled and released from the conducting polymer 115 when the charge of the polymer is altered as a function of oxidation or reduction,

### Definitions used in the description

### Ions

The term "ion" as used herein encompasses not only positively or negatively charged monovalent or multivalent ionic species of atomic elements, but also other molecular species carrying a net positive or negative charge. Hence, in an embodiment of the invention it is intended to transport charged biologically active molecules or macromolecules such as charged amino acids, vitamins, peptides, neurotransmitters, hormones, and substances e.g. pharmaceuticals or endogenous substances. In one embodiment of the invention, the ions that may be transported are cations, for example metal ions, such as potassium or calcium ions. In another embodiment of the invention the ions that may be transported are anions.

### Ionic contact

A first and a second material are in ionic contact when a substantial amount of ions comprised in the first material can move from the first material to the second material, possibly via a third material. The ionic movement may be caused by diffusion or by an applied electric field.

A material which provides an ionic connection between a first and a second material, is a material which is ionically conductive, thus electrically conductive (distinguished from being electronically conductive), and in ionic contact with both said first and said second material.

### Directly or indirectly attached

Two parts which are directly attached to each other are in direct physical contact with each other. With respect to this invention, when a first part is directly attached to a second part, which second part is directly attached to a third part, said first and third parts are referred to as being indirectly attached to each other. Similarly, when said third part is directly attached to a fourth part, said first and fourth parts are referred to as being indirectly attached to each other.

### Semi-solid material

The term semi-solid material refers to a material, which at the temperatures at which it is used has a rigidity and viscosity intermediate between a solid and a liquid. Thus, the material is sufficiently rigid such that it does not flow or leak. Further, particles/flakes in the bulk thereof are substantially immobilized by the high viscosity/rigidity of the material.

In a preferred case, a semi-solid material has the proper rheological properties to allow for the ready application of it on a support as an integral sheet or in a pattern, for example by conventional printing methods. After deposition, the formulation of the material should preferably solidify upon evaporation of solvent or because of a chemical cross-linking reaction, brought about by additional chemical reagents or by physical effect, such as irradiation by ultraviolet, infrared or microwave radiation, cooling etc.

The semi-solid or solidified material preferably comprises an aqueous or organic solvent-containing gel, such as gelatin or a polymeric gel.

### Electrochemically active material

With respect to this invention the term electrochemically active material refers to a material which is capable of being oxidized or reduced when it is in contact with an electrolyte, or another ionically conductive material, and a voltage is maintained across it. Examples of such electrochemically active materials include electrically conductive polymers, as will be described below, and certain metal oxides, such as indium tin oxide (ITO) and tungsten oxide (WO₃).

### Electrolyte

The device may comprise three different electrolytes. It is important to distinguish between the three (source, target, and waste): the source electrolyte contains the ions to be delivered and should not contain a high extent of other ionic species with the same charge (positive or negative) as the ion to be delivered. The target electrolyte may be the body fluid or the cell culture medium, or whatever media needed for the application. Thus, the content of this electrolyte can often not be controlled, but it must be determined from the application of the device. The waste electrolyte serves as a waste for the ions that are transported from the source, and those ions must thus be soluble in this electrolyte.

The electrolyte for use with the device or method of the present invention must be based on a solvent that permits ionic conduction in the electrolyte, i.e. that allows for the dissociation of ionic substances such as salts, acids, bases, etc. The solvent and/or the ionic substance may contribute nucleophiles. Possible electrolytes for use in combination with the inventive device are solutions of salts, acids, bases, or other ion-releasing agents in solvents that support the dissociation of ionic species, thus allowing ionic conductivity. In applications where it is required, the target electrolyte may comprise buffer solutions, such as buffer solutions suitable for use with living organisms or biomolecules, such as proteins. Examples of such buffers include NaHPO₄ and sodium acetate. As other non-limiting examples of possible electrolytes, mention can be made of: aqueous solutions of potassium acetate, calcium acetate, NaCl, Na₂SO₄, H₃PO₄, H₂SO₄, KCl, RbNOs, NH₄OH, CsOH, NaOH, KOH, H₂O₂; organic solvents such as acetonitrile, pyridine, DMSO, DMF, dichloromethane, etc., in combination with suitable salts, such as lithium perchlorate and tertiary ammonium salts, e.g. tetra-butyl ammonium chloride; inorganic solvents such as hypercritical CO₂, liquid SO₂, liquid NH₃, etc., in combination with salts that dissociate in these solvents; solvents displaying auto-dissociation, which results in the formation of ionic species, such as water, formic acid and acetic acid.

The term electrolyte also encompasses solutions comprising charged biologically active molecules or macromolecules such as charged amino acids, proteins, vitamins, peptides or hormones. An electrolyte may also comprise cell culturing media or ingredients thereof, such as proteins, amino acids, vitamins and growth factors.

The electrolyte may also be in a semi-solid or solidified form, preferably comprising an aqueous or organic solvent-containing gel as described above. However, solid polymeric electrolytes are also contemplated and fall within the scope of the present invention. Furthermore, the term electrolytes also encompasses liquid electrolyte solutions soaked into, or in any other way hosted by, an appropriate matrix material, such as a paper, a fabric or a porous polymer.

### Electrodes in the electrolytes

The source, target and optionally the waste electrodes that control the potential of the three respective electrolytes may each comprise a material or a combination of materials which is capable of electron-to-ion conversion, i.e. they need to enable charge transfer between the electrode and its contact and the electrodes must be so called non-polarizable electrodes.

The electrodes of the inventive device may preferably comprise an electrochemically active material, for example Ag/AgCl. Said electrode material may also be an organic material, for example an electrically conductive polymer. Electrically conductive polymers suitable for use in the device of the invention, are preferably selected from the group consisting of polythiophenes, polypyrroles, polyanilines, polyisothianaphthalenes, polyphenylene vinylenes and copolymers thereof such as described by J C Gustafsson et al. in Solid State Ionics, 69, 145-152 (1994); Handbook of Oligo- and Polythiophenes, Ch 10.8, Ed D Fichou, Wiley-VCH, Weinhem (1999); by P Schottland et al. in Macromolecules, 33, 7051-7061 (2000); Technology Map Conductive Polymers, SRI Consulting (1999); by M Onoda in Journal of the Electrochemical Society, 141, 338-341 (1994); by M Chandrasekar in Conducting Polymers, Fundamentals and Applications, a Practical Approach, Kluwer Academic Publishers, Boston (1999); and by A J Epstein et al. in Macromol Chem, Macromol Symp, 51, 217-234 (1991). The the electrically conductive polymer may preferably a polymer or copolymer of a 3,4-dialkoxythiophene, in which said two alkoxy groups may be the same or different or together represent an optionally substituted oxy-alkylene-oxy bridge. It is particularly preferred that the polymer is a polymer or copolymer of a 3,4-dialkoxythiophene selected from the group consisting of poly(3,4-methylenedioxythiophene), poly(3,4-methylenedioxythiophene) derivatives, poly(3,4-ethylenedioxythiophene), poly(3,4-ethylenedioxythiophene) derivatives, poly(3,4-propylenedioxythiophene), poly(3,4-propylenedioxythiophene) derivatives, poly(3,4-butylenedioxythiophene), poly(3,4-butylenedioxythiophene) derivatives, and copolymers therewith.

The electrically conductive polymer may be poly(3,4-ethylenedioxythiophene) (PEDOT). The electrodes may further comprise a polyelectrolyte compound, more preferably said polyelectrolyte compound is polystyrene sulfonic acid) or a salt thereof. One especially preferred material for use in the electrodes of the device of the invention is poly(3,4-ethylenedioxythiophene) with a polystyrene sulfonate) polyanion (in the following referred to as PEDOT:PSS). The electrodes may be present in the form of a thin layer of PEDOT:PSS deposited on a solid substrate.

The electrodes may alternatively be Ag/AgCI, painted on the device, or dipped into the electrolytes.

The source, target and waste electrodes of the inventive device may further comprise a hydrogel. The hydrogel is preferably based on polymers selected from the group consisting of polyacrylates, such as poly(2-hydroxyethyl methacrylate) and poly(acrylamide), polyelectrolytes, such as polystyrene sulfonic acid) (PSS) and poly(acrylic acid) (PAA), polysaccharides, such as agarose, chitosan and dextran, gelatin, polyvinyl alcohol, polyvinyl pyrrolidone and polyethylene glycol.

The electrodes are preferably arranged in a common plane on a solid substrate or dipped into the electrolyte. The electrodes can be additively patterned onto said substrate, e.g., by spincoating, painting, printing, or lamination techniques, or subtractively patterned, *e.g.*, by etching.

At least the target electrode should be biocompatible, since it may be in contact with body fluid or cell culture medium. The term biocompatible is used herein to characterize a material or a surface allowing cultivation of cells thereon or in close association therewith, or the lack of a bodily immunoresponse or similar upon implantation. Cultivation of cells refers to attachment, maintenance, growth and/or proliferation of said cells. An example of an electrode material according to the invention that provides a biocompatible surface is PEDOT:PSS. The biocompatibility of an electrode allow for studies of cellular activities in cells cultivated on or in close association with the electrode.

### The Controlled Delivery Electrode

In order to control the delivery of ions to or from each outlet 109, the device comprises at least one controlled delivery electrode (CDE) 114 which is arranged at or in connection with each delivery site/outlet 109 as illustrated in Fig. 1. The controlled delivery electrode comprises an electronically and ionically conductive material 107, such as an electronically and ionically conductive polymer, and an electrical contact 106. The electronically and ionically conductive material may be PEDOT as described above but also porous metals and supercapacitors.

The electrical contact may be a metal or other conductive material.

The potential from the source to the target is kept constant, but the potential at each controlled delivery electrode may be varied to control the delivery from each outlet respectively.

The electronically and ionically conductive material 107 in combination with a barrier 110 can be seen as an ion diode, where one portion 107 of the diode that is contacted by the electrical contact 106 is electronically as well as cationically (or anionically) conducting. The other part 110 of the diode (the portion that contacts the target electrolyte) is not electronically conducting, but is merely a polycation or polyanion (or any other porous material with fixed charges opposite to the fixed charges of the other half of the diode) and thus acts as a barrier as shown in Fig. 5. The diode does not necessarily consist of two oppositely charged materials, but can also be formed by the conducting polymer/polyelectrolyte blend (or other conducting material with an excess of fixed charges positive or negative) and a small hole with high aspect ratio.

The potential across the controlled delivery electrode and the target electrolyte sets the diode in forward or reverse bias and determines whether ions can pass through it and be delivered to the outlet in the target electrolyte.

### Reference list

100 device or device system
101 source electrolyte
102 target electrolyte
103 ion conductive channel
103' ion conductive channel between the controlled delivery electrodes
104 first electrode or source electrode
105 second electrode or target electrode
106 electrical contact
107 electronically and ionically conductive material
108 electronically and ionically insulating layer
109 delivery site/outlet
110 barrier
110' barrier based on geometry
111 waste channel
112 waste electrolyte
113 waste electrode
114 controlled delivery electrode (CDE) comprising electrical contact 106 and electronically and ionically conductive material 107
115 conducting polymer (in prior art)
201 first end of ion conductive channel 103
202 second end of ion condutive channel 103

## Claims

1. A device (100) comprising a first electrode (104) provided at or in a source electrolyte (101), and at least one ion conductive channel (103), wherein said first electrolyte (101) is arranged at a first portion (201) of the ion conductive channel (103), and a second electrode (105) provided in a target electrolyte (102), wherein said target electrolyte (102) is arranged at a second portion (202) of the ion conductive channel (103), and wherein said first and second electrodes provides an electrical control of an ion flow through the ion conductive channel (103)
c**haracterized** in that
the device further comprises at least one controlled delivery electrode (114) arranged adjacent to or in the second portion of the ion conductive channel (103), wherein said first and second electrodes further are arranged to provide an electrical control of an ion flow through the controlled delivery electrode (114) to the target electrolyte (102), and wherein said controlled delivery electrode (114) is adapted to deliver ions from said ion conductive channel (103) to said target electrolyte (102), and wherein said controlled delivery electrode (114) comprises an electronically and ionically conductive material (107), arranged between said target electrolyte (102) and an electrical contact (106), wherein said controlled delivery electrode (114) is arranged in ionic contact with, and between, said ion conductive channel (103) and the target electrolyte (102), wherein said electrical contact (106) provides for an electrical control potential (V_{CDE}) over the controlled delivery electrode (114) to control an ion flow between the controlled delivery electrode (114) and the target electrolyte (102), when a difference in potential is applied between the controlled delivery electrode (114) and the second electrode (105), such that an internal state of the electronically and ionically conductive material (107) is altered, and wherein a potential (Vₛ) is applied over said first electrode (101) and said second electrode (105).

2. A device (100) as claimed in claim 1, wherein said electronically and ionically conductive material (107) of the controlled delivery electrode (114) is permselective for either cationic or anionic species.

3. The device (100) as claimed in any one of claims 1 or 2, wherein the electronically and ionically conductive material (107) includes a conducting polymer such as PEDOT.

4. The device (100) as claimed in claim 1, wherein said electronically and ionically conducting material (107) is adapted to act as a reservoir for ions being injected to, or extracted from the material.

5. The device (100) as claimed in any one of the preceding claims, wherein said device comprises at least two controlled delivery electrodes (114a, 114b), and wherein said at least two controlled delivery electrodes (114a, 114b) are separated from each other with an ion conductive channel (103') having a finite ion conductivity, to control ion flow from the ion conductive channel (103) into the target electrolyte (102) from each controlled delivery electrode separately.

6. The device (100) as claimed in any one of the preceding claims, wherein a resistance between the controlled delivery electrode (114) and the target electrolyte (102) is sufficiently high to enable the potential drop between the contact (106) of the controlled delivery electrode and the target electrolyte.

7. The device (100) as claimed in claim 5, wherein the controlled delivery electrodes (114a, 114b) are arranged on or in the same or different ion conductive channels (103, 103a, 103b, 103') and wherein the controlled delivery electrodes (114a, 114b) are separated from each other with a material having a finite ion conductivity.

8. The device (100) as claimed in any one of the preceding claims, wherein an ion barrier (110) is arranged between the controlled delivery electrode (114) and the target electrolyte (102).

9. The device (100) as claimed in claim 8, wherein the ion barrier (110) comprises a material having a fixed concentration of opposite charges with respect to the fixed charges of the controlled delivery electrode (114).

10. The device (100) as claimed in any one of claims 8 or 9, wherein the barrier (110') is adapted to geometrically restrain or limit an ion flow.

11. The device (100) as claimed in any one of the preceding claims, wherein the device comprises at least two ion conductive channels (103a, 103b), and wherein multiple controlled delivery electrodes (114a, 114b) are arranged in or on said multiple ion conductive channels (103), wherein said controlled delivery electrodes are in ionic contact with the same target electrolyte (102), and wherein the controlled delivery electrodes (114a, 114b) are separated from each other by an ion conductive channel (103') having a finite ion conductivity.

12. The device (100) as claimed in any one of the preceding claims, wherein the device comprises at least one waste channel (111), and wherein each waste channel comprises a waste electrolyte (112) and a waste electrode (113).

13. A method of operating a device (100), *in vitro,* as claimed in any one of claims 1 to 12, comprising the steps of:
a) providing a source electrolyte (101) comprising the ions to be transported,
b) providing a target electrolyte (102) to where the ions are transported,
c) bringing the source electrode (104) of the device in contact with the source electrolyte,
d) optionally providing a waste electrode (113) and a waste electrolyte (112);
e) bringing the target electrode (105) in contact with the target electrolyte (102);
f) applying a potential to the source electrode (104), the target electrode (105) and the controlled delivery electrode (114), and optionally to the waste electrode (113), effecting ion transport from the source electrolyte (101), to the controlled delivery electrode (114), or optionally to the waste electrolyte (112); and
g) altering the potential of the controlled delivery electrode (114) to switch on ion transport to the target electrolyte (102).

14. Use of a device, *in vitro,* as claimed in any one of claims 1 to 12, for delivering ions from a source electrolyte (101) to a target electrolyte (102) through a controlled delivery electrode (114).

15. Use according to claim 14, wherein said target electrolyte comprises any one of tissue, body fluids or cells *in vitro.*

## Patentansprüche

1. Vorrichtung (100) umfassend eine erste Elektrode (104), die an oder in einem Quellelektrolyten (101) vorgesehen ist, und mindestens einen ionenleitfähigen Kanal (103), wobei der erste Elektrolyt (101) an einem ersten Abschnitt (201) des ionenleitfähigen Kanals (103) angeordnet ist, und eine zweite Elektrode (105), die in einem Zielelektrolyten (102) vorgesehen ist, wobei der Zielelektrolyt (102) an einem zweiten Abschnitt (202) des ionenleitfähigen Kanals (103) angeordnet ist, und wobei die erste und die zweite Elektrode eine elektrische Steuerung eines lonenflusses durch den ionenleitfähigen Kanal (103) bereitstellt,
**dadurch gekennzeichnet, dass**
die Vorrichtung ferner mindestens eine gesteuerte Zuführelektrode (114) umfasst, die neben oder in dem zweiten Abschnitt des ionenleitfähigen Kanals (103) angeordnet ist, wobei die erste und die zweite Elektrode ferner derart angeordnet sind, dass sie eine elektrische Steuerung eines lonenflusses durch die gesteuerte Zuführelektrode (114) zu dem Zielelektrolyten (102) bereitstellen, und wobei die gesteuerte Zuführelektrode (114) derart angepasst ist, dass sie Ionen von dem ionenleitfähigen Kanal (103) zu dem Zielelektrolyten (102) zuführt, und wobei die gesteuerte Zuführelektrode (114) ein elektronisch und ionisch leitfähiges Material (107) umfasst, das zwischen dem Zielelektrolyten (102) und einem elektrischen Kontakt (106) angeordnet ist, wobei die gesteuerte Zuführelektrode (114) in ionischem Kontakt mit und zwischen dem ionenleitfähigen Kanal (103) und dem Zielelektrolyten (102) angeordnet ist, wobei der elektrische Kontakt (106) ein elektrisches Steuerpotenzial (V_{CDE}) über der gesteuerten Zuführelektrode (114) bereitstellt, um einen lonenfluss zwischen der gesteuerten Zuführelektrode (114) und dem Zielelektrolyten (102) zu steuern, wenn eine Potenzialdifferenz zwischen der gesteuerten Zuführelektrode (114) und der zweiten Elektrode (105) angelegt wird, sodass ein innerer Zustand des elektronisch und ionisch leitfähigen Materials (107) verändert wird, und wobei ein Potenzial (V_{S}) über der ersten Elektrode (101) und der zweiten Elektrode (105) angelegt wird.

2. Vorrichtung (100) nach Anspruch 1, wobei das elektronisch und ionisch leitfähige Material (107) der gesteuerten Zuführelektrode (114) entweder für kationische oder anionische Spezies selektiv-permeabel ist.

3. Vorrichtung (100) nach einem der Ansprüche 1 oder 2, wobei das elektronisch und ionisch leitfähige Material (107) ein leitendes Polymer wie PEDOT umfasst.

4. Vorrichtung (100) nach Anspruch 1, wobei das elektronisch und ionisch leitende Material (107) angepasst ist, als Reservoir für Ionen zu dienen, die in das Material eingespeist oder daraus extrahiert werden.

5. Vorrichtung (100) nach einem der vorstehenden Ansprüche, wobei die Vorrichtung mindestens zwei gesteuerte Zuführelektroden (114a, 114b) umfasst, und wobei die mindestens zwei gesteuerten Zuführelektroden (114a, 114b) durch einen ionenleitfähigen Kanal (103') mit einer endlichen lonenleitfähigkeit voneinander getrennt sind, um den lonenfluss von dem ionenleitfähigen Kanal (103) in den Zielelektrolyten (102) von jeder gesteuerten Zuführelektrode separat zu steuern.

6. Vorrichtung (100) nach einem der vorstehenden Ansprüche, wobei ein Widerstand zwischen der gesteuerten Zuführelektrode (114) und dem Zielelektrolyten (102) ausreichend hoch ist, um den Potenzialabfall zwischen dem Kontakt (106) der gesteuerten Zuführelektrode und dem Zielelektrolyten zu ermöglichen.

7. Vorrichtung (100) nach Anspruch 5, wobei die gesteuerten Zuführelektroden (114a, 114b) an oder in den gleichen oder verschiedenen ionenleitfähigen Kanälen (103, 103a, 103b, 103') angeordnet sind, und wobei die gesteuerten Zuführelektroden (114a, 114b) durch ein Material mit einer endlichen lonenleitfähigkeit voneinander getrennt sind.

8. Vorrichtung (100) nach einem der vorstehenden Ansprüche, wobei eine lonenbarriere (110) zwischen der gesteuerten Zuführelektrode (114) und dem Zielelektrolyten (102) angeordnet ist.

9. Vorrichtung (100) nach Anspruch 8, wobei die lonenbarriere (110) ein Material mit einer feststehenden Konzentration entgegengesetzter Ladungen in Bezug auf die feststehenden Ladungen der gesteuerten Zuführelektrode (114) umfasst.

10. Vorrichtung (100) nach einem der Ansprüche 8 oder 9, wobei die Barriere (110') ausgelegt ist, einen lonenstrom geometrisch einzuschränken oder zu begrenzen.

11. Vorrichtung (100) nach einem der vorstehenden Ansprüche, wobei die Vorrichtung mindestens zwei ionenleitfähige Kanäle (103a, 103b) umfasst, und wobei mehrere gesteuerte Zuführelektroden (114a, 114b) in oder an den mehreren ionenleitfähigen Kanälen (103) angeordnet sind, wobei die gesteuerten Zuführelektroden in ionischem Kontakt mit dem gleichen Zielelektrolyten (102) stehen, und wobei die gesteuerten Zuführelektroden (114a, 114b) durch einen ionenleitfähigen Kanal (103') mit einer endlichen lonenleitfähigkeit voneinander getrennt sind.

12. Vorrichtung (100) nach einem der vorstehenden Ansprüche, wobei die Vorrichtung mindestens einen Ausschusskanal (111) umfasst, und wobei jeder Ausschusskanal einen Ausschusselektrolyten (112) und eine Ausschusselektrode (113) umfasst.

13. Verfahren zum Betreiben einer Vorrichtung (100) *in vitro* nach einem der Ansprüche 1 bis 12, das die Schritte umfasst:
a) Bereitstellen eines Quellelektrolyten (101), der die zu transportierenden Ionen umfasst,
b) Bereitstellen eines Zielelektrolyten (102), zu dem die Ionen transportiert werden,
c) Inkontaktbringen der Quellelektrode (104) der Vorrichtung mit dem Quellelektrolyten,
d) optionales Bereitstellen einer Ausschusselektrode (113) und eines Ausschusselektrolyten (112);
e) Inkontaktbringen der Zielelektrode (105) mit dem Zielelektrolyten (102);
f) Anlegen eines Potenzials an die Quellelektrode (104), die Zielelektrode (105) und die gesteuerte Zuführelektrode (114) und optional an die Ausschusselektrode (113), wodurch ein Ionentransport von dem Quellelektrolyten (101) zu der gesteuerten Zuführelektrode (114) oder optional zu dem Ausschusselektrolyten (112) bewirkt wird; und
g) Ändern des Potenzials der gesteuerten Zuführelektrode (114), um einen Ionentransport zu dem Zielelektrolyten (102) einzuschalten.

14. Verwendung einer Vorrichtung *in vitro* nach einem der Ansprüche 1 bis 12 zum Zuführen von Ionen von einem Quellelektrolyten (101) zu einem Zielelektrolyten (102) durch eine gesteuerte Zuführelektrode (114).

15. Verwendung nach Anspruch 14, wobei der Zielelektrolyt eines von Gewebe, Körperflüssigkeiten oder Zellen *in vitro* umfasst.

## Revendications

1. Dispositif (100) comprenant une première électrode (104) disposée au niveau de ou dans un électrolyte source (101), et au moins un canal conducteur ionique (103), dans lequel ledit premier électrolyte (101) est agencé au niveau d'une première partie (201) du canal conducteur ionique (103), et une deuxième électrode (105) prévu dans un électrolyte cible (102), dans lequel ledit électrolyte cible (102) est agencé au niveau d'une deuxième partie (202) du canal conducteur ionique (103), et dans lequel lesdites première et deuxième électrodes fournissent une commande électrique d'un flux d'ions à travers le canal conducteur ionique (103), **caractérisé en ce que** le dispositif comprend en outre au moins une électrode de distribution contrôlée (114) agencée à côté ou dans la deuxième partie du canal conducteur ionique (103), dans lequel lesdites première et deuxième électrodes sont en outre agencées pour fournir une commande électrique d'un flux d'ions à travers l'électrode de distribution contrôlée (114) vers l'électrolyte cible (102), et dans lequel ladite électrode de distribution contrôlée ( 114) est adapté pour fournir des ions dudit canal conducteur ionique (103) audit électrolyte cible (102), et dans lequel ladite électrode de distribution contrôlée (114) comprend un matériau conducteur électronique et ionique (107), disposé entre ledit électrolyte cible (102) et un contact électrique (106), dans lequel ladite électrode de distribution contrôlée (114) est disposée en contact ionique avec et entre ledit canal conducteur ionique (103) et l'électrolyte cible (102), dans lequel ledit contact électrique (106) fournit un potentiel de commande électrique (V_{CDE}) sur l'électrode de distribution contrôlée (114) pour contrôler un flux d'ions entre l'électrode de distribution contrôlée (114) et l'électrolyte cible (102), lorsqu'une différence de potentiel est appliquée entre l'électrode de distribution contrôlée (114) et la deuxième électrode (105), de telle sorte qu'un état interne du matériau conducteur électroniquement et ioniquement (107) est modifié, et dans lequel un potentiel (Vₛ) est appliqué sur ladite première électrode (101) et ladite deuxième électrode (105).

2. Dispositif (100) selon la revendication 1, dans lequel ledit matériau électroniquement et ioniquement conducteur (107) de l'électrode de distribution contrôlée (114) est permsélectif pour les espèces cationiques ou anioniques.

3. Dispositif (100) selon une quelconque des revendications 1 ou 2, dans lequel le matériau conducteur électronique et ionique (107) comprend un polymère conducteur tel que le PEDOT.

4. Dispositif (100) selon la revendication 1, **caractérisé en ce que** ledit matériau conducteur électronique et ionique (107) est adapté pour servir de réservoir d'ions injectés ou extraits du matériau.

5. Dispositif (100) selon une quelconque des revendications précédentes, dans lequel ledit dispositif comprend au moins deux électrodes de distribution contrôlée (114a, 114b), et dans lequel lesdites au moins deux électrodes de distribution contrôlée (114a, 114b) sont séparées l'un de l'autre par un canal conducteur ionique (103') ayant une conductivité ionique finie, pour contrôler le flux d'ions du canal conducteur ionique (103) dans l'électrolyte cible (102) à partir de chaque électrode de livraison séparément.

6. Dispositif (100) selon une quelconque des revendications précédentes, dans lequel une résistance entre l'électrode de distribution contrôlée (114) et l'électrolyte cible (102) est suffisamment élevée pour permettre la chute de potentiel entre le contact (106) de l'électrode de distribution contrôlée et l'électrolyte cible.

7. Dispositif (100) selon la revendication 5, dans lequel les électrodes de délivrance contrôlée (114a, 114b) sont disposées sur ou dans le même ou différents canaux conducteurs ioniques (103, 103a, 103b, 103') et dans lequel les électrodes de distribution contrôlée (114a, 114b) sont séparées les unes des autres par un matériau ayant une conductivité ionique finie.

8. Dispositif (100) selon une quelconque des revendications précédentes, dans lequel une barrière ionique (110) est agencée entre l'électrode de distribution contrôlée (114) et l'électrolyte cible (102).

9. Dispositif (100) selon la revendication 8, dans lequel la barrière ionique (110) comprend un matériau ayant une concentration fixe de charges opposées par rapport aux charges fixes de l'électrode de distribution contrôlée (114).

10. Dispositif (100) selon une quelconque des revendications 8 ou 9, dans lequel la barrière (110') est adaptée pour restreindre ou limiter géométriquement un flux d'ions.

11. Dispositif (100) selon une quelconque des revendications précédentes, dans lequel le dispositif comprend au moins deux canaux conducteurs ioniques (103a, 103b), et dans lequel de multiples électrodes de distribution contrôlée (114a, 114b) sont agencées dans ou sur lesdits multiples canaux conducteurs ioniques (103), dans lequel lesdites électrodes de distribution contrôlée sont en contact ionique avec le même électrolyte cible (102), et dans lequel les électrodes de distribution contrôlée (114a, 114b) sont séparées les unes des autres par un canal conducteur ionique (103') ayant une conductivité ionique finie.

12. Dispositif (100) selon une quelconque des revendications précédentes, dans lequel le dispositif comprend au moins un canal de rebuts (111), et dans lequel chaque canal de rebuts comprend un électrolyte de rebuts (112) et une électrode de rebuts (113).

13. Procédé de fonctionnement d'un dispositif (100), in vitro, selon une quelconque des revendications 1 à 12, comprenant les étapes consistant à
a) fournir un électrolyte source (101) comprenant les ions à transporter,
b) fournir un électrolyte cible (102) à l'endroit où les ions sont transportés,
c) mettre en contact l'électrode source (104) du dispositif avec l'électrolyte source,
d) fournir facultativement une électrode de rebut (113) et d'un électrolyte de rebut (112) ;
e) amener l'électrode cible (105) en contact avec l'électrolyte cible (102) ;
f) appliquer un potentiel à l'électrode source (104), à l'électrode cible (105) et à l'électrode de distribution contrôlée (114), et facultativement à l'électrode de rebut (113), effectuant le transport des ions depuis l'électrolyte source (101), à l'électrode de distribution contrôlée (114), ou éventuellement à l'électrolyte de rebut (112) ; et
g) modifier le potentiel de l'électrode de distribution contrôlée (114) pour activer le transport d'ions vers l'électrolyte cible (102).

14. Utilisation d'un dispositif, in vitro, selon une quelconque des revendications 1 à 12, pour délivrer des ions d'un électrolyte source (101) à un électrolyte cible (102) à travers une électrode de distribution contrôlée (114).

15. Utilisation selon la revendication 14, dans laquelle ledit électrolyte cible comprend un quelconque des tissus, fluides corporels ou cellules in vitro.
